# EUROPEAN PATENT SPECIFICATION

(11) **EP 1 593 425 B1**
(45) Date of publication and mention of the grant of the patent: **09.08.2006**
(21) Application number: 04447111.8
(22) Date of filing: 04.05.2004
(51) Int. Cl.: B01J 19/00, C12Q 1/68, C07H 21/00

(54) **Preparation method and use of micro-arrays supports for detection of multiple polynucleotide sequences with high sensitivity**
Herstellungsverfahren und Verwendung von Mikro-Array-Trägern zur Detektion mehrfacher Polynukleotidsequenzen mit hoher Empfindlichkeit
Procédé de préparation et utilisation de supports de microréseaux pour la détection de séquences polynucléotidiques multiples à haute sensibilité

(43) Date of publication of application: 09.11.2005
(73) Proprietor: Eppendorf Array Technologies SA, B-5000 Namur (BE)
(72) Inventor: Remacle, José, 5020 Malonne (BE); Beyreuther, Konrad, 69120 Heidelberg (DE)
(74) Representative: Van Malderen, Joëlle

(56) References cited:
- WO-A-02/04111
- WO-A-99/66076
- WO-A-03/040342
- TAKAHASHII K ET AL: "A photochemical/chemical direct method of synthesizing high-performance deoxyribonucleic acid chips for rapid and parallel gene analysis" SENSORS AND ACTUATORS B, ELSEVIER SEQUOIA S.A., LAUSANNE, CH, vol. 83, no. 1-3, 15 March 2002 (2002-03-15), pages 67-76, XP004344487 ISSN: 0925-4005
- ZAMMATTEO NATHALIE ET AL: "Comparison between different strategies of covalent attachment of DNA to glass surfaces to build DNA microarrays" ANALYTICAL BIOCHEMISTRY, ACADEMIC PRESS, SAN DIEGO, CA, US, vol. 280, no. 1, 10 April 2000 (2000-04-10), pages 143-150, XP002159089 ISSN: 0003-2697
- BEAUCAGE S L: "STRATEGIES IN THE PREPARATION OF DNA OLIGONUCLEOTIDE ARRAYS FOR DIAGNOSTIC APPLICATIONS" CURRENT MEDICINAL CHEMISTRY, BENTHAM SCIENCE PUBLISHERS BV, BE, vol. 8, no. 10, August 2001 (2001-08), pages 1213-1244, XP009030910 ISSN: 0929-8673
- MCQUAIN M K ET AL: "Chaotic mixer improves microarray hybridization" ANALYTICAL BIOCHEMISTRY, ACADEMIC PRESS, SAN DIEGO, CA, US, vol. 325, no. 2, 15 February 2004 (2004-02-15), pages 215-226, XP004486078 ISSN: 0003-2697
- MATYSIAK S ET AL: "IMPROVED SOLID SUPPORTS AND SPACER/LINKER SYSTEMS FOR THE SYNTHESIS OF SPATIALLY ADDRESSABLE PNA-LIBRARIES" NUCLEOSIDES & NUCLEOTIDES, MARCEL DEKKER, INC, US, vol. 18, no. 6/7, 1999, pages 1289-1291, XP009021993 ISSN: 0732-8311

## Description

### Field of the invention

The present invention relates to a new preparation method of micro-arrays, to the obtained micro-arrays supports, to the kits comprising them and to their use for the detection with high efficiency of multiple target polynucleotides (or nucleotide sequences) present in a biological sample or in a test solution. The obtained micro-arrays supports are particularly useful in molecular diagnosis and gene expression analysis.

The micro-arrays supports of the present invention also allows the identification, detection and/or quantification of a large number of genes or gene products (amplicons) obtained by genetic amplification, these genes or gene products being present in a sample and are obtained from (micro)organisms comprising said genes that belong to different genetical taxonomic groups (class, family, genus, species, individuals).

### Background of the invention

Identification of multiple expressed genes and identification of (micro-)organisms can be done through the detection of specific sequences from a genetic material. Identification and quantification of expressed genes (present in a sample) is usually performed after reverse transcribing mRNA into its corresponding cDNA and detection of said cDNA. Detection of specific (micro)organisms is performed easily by amplifying (or copying) a particular sequence of a genomic DNA (present in a sample) and then detecting and/or identifying the amplified sequences. In both cases, the amplified or copied sequences (target sequences) can be detected via specific hybridization upon corresponding capture molecules present on or attached to a solid support according to micro-arrays. Quantification of the target sequences bound to their specific capture molecules allows estimation of the amount of mRNA or genomic DNA present in the sample. Preferably, appropriate control means are included and the necessary corrections made to take into account the efficiency of the different steps, such as the copying or amplification steps of target sequences and their binding via hybridization upon capture molecules forming a micro-array.

Micro-arrays are solid supports being produced mainly according to two methods. The first method, described in US patents 5,510,270 and 5,700,637, is based on photolithographic *in situ* synthesis of capture sequences on a solid support surface. Photolithographic DNA synthesis uses rapid solid phase phosphoramidite chemistry. Positional and sequential control are achieved by a combination of 5'-photoprotected phosphoramidites, which can be activated by irradiation with light, and a set of masks containing holes at appropriate positions through which light can pass. Upon excitation, the photoprotecting groups present on partial oligonucleotide sequences, synthesized earlier during the process, are removed and the oligomers are extended by another nucleotide after adding the relevant monomer. Current coupling efficiencies impose an actual size limit of about 25 bases to these micro-arrays. Beyond this limit, incomplete products accumulate which impair the specificity of the assay.

The limitation in size or length of capture oligonucleotides according to this method, is due to the fact that the efficiency of synthesis performed step by step is not 100% so that accumulation of truncated oligonucleotides occurs within the same spot. The photolithographic method results in the presence of short oligonucleotides on a support. The main advantage of the method is the possibility to miniaturize the thus obtained of capture nucleotide sequences and to generate high-density arrays containing several thousands of capture nucleotide sequences.

The second method is based on the chemical or enzymatic synthesis of capture sequences before mechanical deposition onto known specific locations (according to a solid support surface). With this method, there is no restriction in the size of the sequences to be spotted, deposited or attached since they can be synthesized according to any chemical or enzymatic method and their sequences confirmed by an analytical method (usually a sequencing method). A major advantage of the deposition technology, compared to the *in situ* synthesis approach, is its great versatility. This method allows production of micro-arrays with virtually any capture molecule of interest, including but not limited to nucleic acid sequences of any length, antibodies, lipids, carbohydrates, small chemical compounds, etc. Furthermore, the synthesis of sequences can be optimized, sequences can be purified, their quality checked before use and/or their concentration adjusted before coupling to the solid surface. However, one disadvantage of the method is that the process is time-consuming since each capture molecule sequence has to be handled separately before spotting on the solid support surface according to an array, thereby limiting the size of the obtained micro-arrays.

The chemistry of a hybridization-to-oligonucleotide micro-array is clearly different from that of an array constructed with long DNA capture sequences or molecules. It has been observed that long specific capture sequences give much better binding of a complementary target sequence present in a solution or sample than their corresponding short fragments. In practice long polynucleotide capture sequences are used for direct binding of long polynucleotide target sequences. In a typical gene expression experiment, the capture nucleotides sequences for cDNA binding contain 50 bases or more, for example 70 bases, or may even contain 600 bases or nucleotides.

When short oligonucleotides of 15-20 bases only are used as capture sequences (see e.g. US 5,510,270), binding of long cDNA is scarce and possibly not detectable. For adequate detection, identification and/or quantification of long cDNAs, the following additional steps need to be taken. The RNA sequences are first reverse transcribed into corresponding cDNA by using a primer carrying a transcription start site for T7 RNA polymerase. These cDNA sequences are then retranscribed in vitro into several RNA copies which are then cut into small pieces. These small RNA fragments are then used for hybridization on arrays bearing a series of capture sequences for each of these RNA fragments. Fragmentation is necessary to ensure sufficient access of the target RNA sequences to the very short capture sequences. Specific algorithms are required to adequately correlate the hybridization pattern of these different capture molecules with the original sequence(s) of the target DNA or mRNA. To be able to control specific hybridization of a particular target sequence, it is also necessary to perform for each capture nucleotide sequence a control, which consists into the addition of a control capture nucleotide sequence being identical to the capture nucleotide sequence but with one base difference.

Another problem arises with double stranded DNA (dsDNA). When dsDNA is to be analyzed on a micro-array, it will preferentially re-associate in solution rather than being hybridized upon corresponding capture nucleotide sequences present on or attached to a solid substrate surface. The use of short oligonucleotides for direct identification and/or quantification of large double stranded amplicons (obtained after genetic amplification) results in very low or no sensitivity at all, and is consequently of no practical use. Again the amplicons have to be retranscribed into RNA using a double amplification process performed with primer(s) bearing T3 or T7 sequences and then a retrotranscription with a RNA polymerase. These RNAs are cut into pieces of about 40 bases before being detected on an array (see e.g. example 1 of international patent application WO97/29212). The above technique was herein applied for the identification of the *Mycobacterium tuberculosis rpoB* gene, using capture nucleotide sequences of less than 30 nucleotides. The described method is complicated in the sense that it does not allow direct detection of amplicons resulting from genetic amplification reactions (such as PCR), but requires another cycle of reactions and copying of target sequences, which each introduce extra bias in the quantification of said target sequences.

The construction of micro-arrays via chemical synthesis and deposition of short polynucleotide sequences, is however useful, since it is a fast and low-price process. In addition to that, the design of capture molecules or sequences can be easily adapted according to the requirements of the sequences to be analyzed or discriminated. However, as explained here above the use of short nucleotide capture probes leads to strong limitation in the detection of long target nucleotide fragments. WO02/04111 discloses a solid support comprising a nucleotide hooks and lines, said nucleotide line being non random poly T decamer.

### Aims of the invention

The present invention aims to provide a novel production method for obtaining micro-array supports that do not have the drawbacks of the prior art.

The present invention in particular aims to provide a novel method for the fast construction of micro-array supports bearing sequences that advantageously combine in a detection method (1) the high specificity of short nucleotide sequences specific to target nucleotide sequences to be detected, identified and/or quantified with (2) the high sensitivity of long nucleotide sequences (same binding sensitivity as long nucleotide sequences of over 150 bases).

Another aim of the invention is to provide novel (micro) arrays supports that are highly suited for the direct detection of cDNAs or of long double stranded DNA sequences.

A further aim of the invention is to provide novel construction methods to obtain standard (micro)array solid supports that are highly suited for further adaptation and construction of customizable (micro)arrays adaptable to the needs of different consumers.

### Summary of the invention

A first aspect of the present invention concerns a new method for obtaining (micro)arrays of nucleotide sets on a surface of a solid support, to be used for the detection, identification and/or the quantification of at least two, preferably at least four, different target polynucleotides or nucleotide sequences, possibly present in a biological sample or test solution.

Advantageously, said method comprises the steps of:
- fixing nucleotide line sequences upon a surface of a solid support, said nucleotide lines being at least 20 nucleotides long and having a (random) nucleotide sequence non related to target nucleotide sequences to be detected and/or quantified (which means that said sequences are not able to complementary hybridize with said target nucleotide sequences);
- deposing, in at least 4 specific locations on the surface of the solid support covered by said nucleotide lines, nucleotide hooks having a sequence specific for a target polynucleotide sequence to be detected and/or quantified and;
- covalently linking at said specific surface locations the nucleotide hooks to the nucleotide lines, in order to form polynucleotide sets, specific for the binding of said target polynucleotides or nucleotide sequences at said specific locations of the solid support surface.

The nucleotide lines are sequences fixed by a covalent bond on the surface of the solid support by one of their extremities (5' or 3') and are able to fix the nucleotide hooks by the other unbound extremity.

The obtained array with long nucleotide sets construct in such simple and non expensive method provides both (1) the high specificity of the short specific sequence of the nucleotide hooks, each nucleotide hook being specific for one target sequence among the at least four different polynucleotides or nucleotide sequences, and (2) a high sensitivity reaching the advantageous sensitivity of long polynucleotide sequences of over 150 bases, possibly over 350 bases.

Furthermore, a preferred method aims to obtain an uniform and efficient covering of the surface of the solid support by deposit of the nucleotide lines, that allows a further covalent linking of nucleotide hooks to these nucleotide lines, at a specific location on the surface of the solid support. Not all the said surface of the solid support results in the formation of polynucleotide sets, because some locations of the said solid support surface remain covered only by nucleotide lines.
Possibly, the line nucleotide sequences are fixed onto the surface of the solid support through an adaptor molecule being for instance streptavidin, antigen or antibody (that combined to a complementary molecule (Biotin, Antibody, Antigen) already fixed upon the solid support surface).
As such, it is also possible by the method according to the invention to select only specific locations on the surface of the solid support which comprise similar or different linked (to the lines) nucleotide hooks able to bind to the same or different targets polynucleotides or nucleotide sequences to be detected, identified and/or quantified.

The solid support surface of the invention preferably comprises a micro-array of at least four spots of polynucleotide sets per cm².

The nucleotide lines sequences of such array can be one random (the same random) sequence or can be multiple random (different random) sequences. Preferably, said nucleotide lines do not comprise or are not made of sequences which are able to hybridize, under the conditions used, with any of the target polynucleotides or nucleotide sequences to be detected and/or quantified possibly present in a biological sample or test solution.

Hybridization of two nucleotide strands, as known by the person skilled in the art, is defined by the percentage of identity or similarity of the two sequences and is defined by the hybridization conditions used (e.g. stringent, less stringent or non-stringent conditions).
In the present case, the meaning of the term "do not hybridize" implies that there will be less than 1% of target hybridized on the capture polynucleotides and preferably less than 0.1% and even preferably less than 0.01%. To avoid hybridization, there will be no more than around 15 consecutive complementary base pair bindings between a target polynucleotide (or nucleotide) sequence and its specific or corresponding nucleotide line sequence, preferably there will be less than ten such pairings possible, more preferably less than five. As such, the sequences of the nucleotide lines will contain, preferably less than fifteen bases and more preferably, less than ten and still more preferably less than five contiguous bases complementary to the target sequences to be detected. The determination of possible consecutive sequences is easily done by comparison of the sequences to molecular database as provided by Genbank and using software such as Nucleotide-nucleotide BLAST (blastn) (http://www.ncbi.nlm.nih.gov/BLAST).

According to another embodiment of the present invention, the nucleotide lines are obtained by an *in situ* synthesis of nucleotide sequences on the support.

According to a further preferred embodiment of the present invention, the nucleotide lines have a sequence comprised of between about 20 and about 1000 nucleotides, more preferably between about 50 and about 200 nucleotides, and even more preferably between about 60 and about 100 nucleotides.

According to another embodiment of the present invention, the nucleotide lines coupled to the nucleotide hooks are sequences having different binding affinities and attached to different solid supports, each of these solid supports being characterized by a specific chemical or physical condition or feature, such as a specific chemical or physical binding affinity, (i.e.: different beads of different chemical of physical nature including (but not limited to) different size, fluorescence, color, magnetism, etc). This technical feature allows a discrimination of the sequences by an analysis of the chemical or physical nature of the solid support upon which they are bound.

According to the invention, the target polynucleotides or nucleotide sequences to be detected, identified and/or quantified are DNA or RNA sequences such as genomic DNA or amplicons, possibly amplified or copied before their binding upon the nucleotide hooks.

Another aspect of the present invention is related to the micro-arrays obtainable by the method according to the invention for the detection, identification and/or the quantification of at least 2, at least 4, preferably at least 10, more preferably at least 30 different target polynucleotides or nucleotide sequences, possibly simultaneously present in a biological sample or test solution.

Another aspect of the present invention is related to a solid support comprising nucleotide lines having a random sequence of at least 20 nucleotides long and being uniformly covalently fixed by a first extremity of their sequences upon the surface of the solid support, surface being used for the preparation of micro-array according to the invention. Said "treated" solid support can be used directly by the consumer as an intermediate product adapted for the preparation of detection micro-arrays solid supports; the consumer being able to form polynucleotide sets after the addition of nucleotide hooks (to be linked to the nucleotide lines) at specific locations of the "treated" solid support surface.
This solid support is defined hereafter as a "customizable micro-array".

Advantageously, the other (free) extremity (not fixed to the surface) of the nucleotide line sequences preferably comprise a reactive group or function that can thereafter react covalently (to form a link) with (possibly functionalised) nucleotide hook sequences.

Preferably, said nucleotide hooks are also sequences terminated by one or more reactive chemical groups of functions that can react (to form a link) with the nucleotide lines(functionalized or not). Preferably, said reactive chemical functions or groups of the nucleotide line extremity are made of aldehyde, epoxide or acrylate functions that can react directly with the NH₂ function of a nucleotide hook extremity. In a preferred embodiment, the nucleotide lines fixed on the support contain at (or near (which means a distance that allows a binding with the extremity of another nucleotide sequence (hook sequence)) their free extremity a nucleotide ribose. The ribose is further oxydized into aldehydes for the consecutive fixation of NH₂ function of a nucleotide hooks extremity.

Advantageously, the density of the polynucleotide lines and/or the polynucleotide sets upon the surface of the solid support is superior to 10 fmoles, preferably 100 fmoles/cm² of solid support surface.

In another preferred embodiment, the covalent link between the line sequences and the hook sequences is not a phosphodiester link.

Another aspect of the present invention is related to the said solid support wherein the fixed nucleotide lines with a functionalised sequence extremity bound to nucleotide hooks by a covalent link forming polynucleotide sets wherein said solid support surface comprises bound nucleotide hooks to nucleotide lines only at specific locations of this solid support surface and wherein other locations are still covered only by the nucleotide lines.
This means that the solid support surface comprises two types of fixed nucleotide sequences ; polynucleotides sets upon specific locations of the solid support and nucleotide lines preferably functionalised with reactivated chemical groups or functions upon other locations of the said solid support surface. Other locations are those available for further fixation of hooks. This type of solid support is defined hereafter as a "customized micro-array" or "semi-customized micro-array".

This means that this type of "customized micro-array" comprises specific locations upon the surface of the solid support that can be modified by the consumer in order to form other polynucleotide sets after the addition of specific nucleotide hooks (selected by the consumer) upon the nucleotide lines at specific locations of the solid support surface, said nucleotide hooks being able to react covalently with the functionalised extremity of the nucleotide lines.
In another aspect, the "semi-customized micro-array" also includes solid support surface comprising two types of fixed nucleotide sequences; standard polynucleotides upon specific locations of the solid support not covered by nucleotide lines (according to an array of the prior art) and nucleotide lines upon other locations of said solid support. This means that this type of customized micro-array comprises conventional polynucleotides prespotted on specific locations of the solid support and nucleotide lines upon other locations of said solid support that can be modified by the consumer in order to form polynucleotide sets after the addition of specific nucleotide hooks at specific locations of the solid support surface covered by nucleotide lines.

Another aspect of the present invention is related to a kit of parts comprising the solid support according to the invention (customisable micro-array or customised micro-array) means and media selected for obtaining adapted micro-array for a consumer. Such means and media are preferably chemical groups or functions that can react with the extremity of nucleotide lines or nucleotide hooks, nucleotide hooks (preferably functionalised) that can react with functionalised nucleotide lines, as well as media (buffer) which could be used for washing the surface of the solid support in order to remove unbound nucleotide hooks sequences.

The nucleotide hooks (preferably functionalised nucleotide hooks) present in the kit of parts according to the invention are chosen according to the customer application need. Therefore, this kit of parts is flexible and adjustable to a requested detection, identification and/or quantification of multiple target molecules in a sample. This means that the "customised micro-array" according to the invention can already comprise polynucleotide sets with specific nucleotide hooks at specific locations of the solid support surface, said polynucleotide sets being present at said specific locations according to an array and their location information is provided to the customer in order to allow him to complete the preparation of said "customised micro-array" with additional sequences at other specific locations for improving a requested detection, identification and/or quantification of multiple target molecules.

The means and media present in the kit of parts are also selected for performing one or more genetic amplification or copy steps and for obtaining a specific detection, identification and/or quantification of multiple nucleotide sequences of interest. Such means and media well known by the person skilled in the art include primers, cycler, polymerase or other media, such as fluorescence, colorimetric and/or radioactive labels used for obtaining a signal resulting from the binding of target nucleotide sequences upon their corresponding nucleotide sets.

Detection, identification and/or quantification of multiple target molecules can be done via the naked eye, but preferably is done in special apparatuses such as automatic readers, preferably equipped with the necessary software for detection and/or interpretation of signals that are generated resulting from the binding of target molecules to their respective capture molecules (hooks extremity of polynucleotide sets). Such apparatuses can be fully automated.

In the context of the present invention, the meaning of the terms "nucleic acid", "oligonucleotide", "target or nucleotide sequences", "array", "nucleotide sequence", "target nucleic acid", "bind substantially", "hybridizing specifically to", "background", "quantifying" and the like is as described in the international patent application WO97/27317, The term "polynucleotide" in the present context refers to nucleotide sequences or nucleotide like sequences of at least 2 nucleotides or nucleotide analogues. The term "oligonucleotide" or "short polynucleotide" refers in particular to nucleotide or nucleotide like sequences of less than 100 and preferably less than 50 nucleotides or nucleotide anologues. The term "long polynucleotide" refers to nucleotide or nucleotide like sequences of more than 100 nucleotides. References to nucleotide(s), oligonucleotide(s) and the like include analogous species wherein the sugar-phosphate backbone is modified and/or replaced, provided that its hybridization properties are not impaired. By way of example the backbone may be replaced by an equivalent synthetic peptide, called Peptide Nucleic Acid (PNA) or by Intercalating Nucleic Acid (INA).

The meaning of the terms "homologous sequence(s)", and "homologues" is as described in European patent EP 1266034.

The term "solid support" for building micro-arrays in the present context is meant to comprise any type of solid material which can be physically handled to perform the necessary reactions like incubation of a test solution or sample possibly containing target nucleotide sequence or copies of a target nucleotide sequence. This solid support can be unique or can be a composite made of several materials, one of them being a substrate on which sequences are fixed to yield a micro-array. Typical examples of substrates used in the field are polymers which may be functionalised in order to better fix the capture molecules. Said substrates or supports on which the nucleotide lines are fixed may be a porous or non porous support, may be smooth or rough and may be deposed or placed on top of another solid support, being made for example of glass or of plastic.
The terms "sample", "biological sample" or "test solution" are meant to comprise any sample or solution suspected to contain a target nucleotide sequence. In the context of the present invention, especially polynucleotides or nucleotide sequences specific to a (micro) organism or to a component thereof are being detected. A test solution may be a solution containing amplified products amplicon of a (micro) organism or a component thereof.
The term "uniformly" is meant to comprise homogenous layer of nucleotide lines fixed upon the surface of the solid support. In this context, the density of nucleotide lines per cm² of surface is uniform and differs by less than 30% and preferably by less than 10% in the different parts of the surface of the solid support. The fixation of the nucleotide hooks has to be quantitative and similar in the different spots of the array because nucleotide hooks are the capture molecules for the multiple target nucleotides being present in the same sample and for which the amount has to be quantified and compared to each other.

The invention will be described in further details in the following examples and specific embodiments, by reference to the enclosed drawings. The examples, embodiments and drawings are not in any way intended to limit the scope of the invention as claimed, neither are the reference signs used.

### Description of the figures

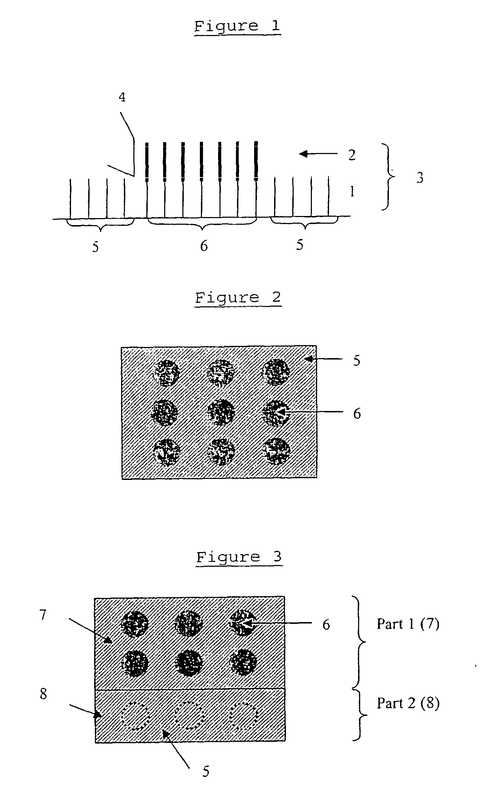

The figure 1 presents an array according to the invention with nucleotide lines (1), nucleotide hooks (2) and polynucleotide sets (3) for the specific detection, identification and/or quantification of multiple target polynucleotides or nucleotide sequences (4) in a sample on specific locations (6) on a surface (5) of a solid support covered with nucleotide lines (1).

The figure 2 presents the surface of an array (5) according to the invention covered by lines (1) or (line-hook) polynucleotide specific sets (3) present in specific location (6). The surface of the solid support used for the construction of the (micro)-array is uniformly covered with nucleotide lines (1).

The figure 3 presents the surface of a "semi-customized array" according to the invention covered on one part (7) with polynucleotide sets specific of some target sequences present on specific locations of the support (6) and another part (8) of the surface (5) covered with nucleotide lines (1) for further nucleotide hooks (2) binding in order to provide specific polynucleotide sets (3) for further target detection.

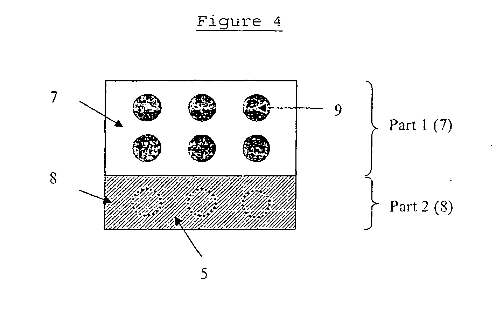

The figure 4 presents the surface of a "semi-customized array" according to the invention covered on one part (7) with polynucleotides specific of some target sequences present on specific locations of the support (9) not covered by nucleotide lines (1) and another part (8) of the surface (5) covered with nucleotide lines (1) for further nucleotide hooks (2) binding in order to provide specific polynucleotide sets (3) for further target detection.

### Detailed description of the invention

The present invention relates to a novel construction method resulting in novel (micro)-arrays highly suited for the detection of multiple target nucleotide sequences possibly present in a biological sample or test solution, in particular cDNA sequences or long double stranded DNA sequences. In an embodiment according to the invention, at least part of the solid support surface (5) of the array obtained as such is covered, possibly at random, with nucleotide sequences serving as nucleotide lines (1), on which at specific locations (6) specific nucleotide hooks (2) are then deposited to yield polynucleotide sets (3) which allow specific binding of corresponding target nucleotide sequences. The finally bound sequence (also referred to as the polynucleotide set (3)) is a long nucleotide sequence or polynucleotide having for one part a non specific sequence bound to the support and for another part a sequence that specifically recognizes or binds the target sequence (4) to be detected (see Figure 1 and Figure 2). Said polynucleotide sets (3) are also referred to as capture sequences, specific capture sequences or corresponding capture sequences, meaning that they recognize a target nucleotide sequence with part of its sequence complementary to the target nucleotide sequence.

In a preferred embodiment of the invention, the nucleotide lines (1) are present on the entire surface (5) of the substrate or micro-array support that is in contact with the sample. This surface is then partly or totally covered with specific nucleotide hooks (2).

In a particular embodiment of the invention, all nucleotide lines (1) may consist of the same random sequence which is not complementary to the target organism genome on more than 10 consecutive bases.

In another embodiment of the invention, the line nucleotide sequences (1) are not identical, i.e. the nucleotide lines do not all have the same sequence but cover a plurality of different sequences. The sequences of the nucleotide lines may be known or unknown.

In a particular embodiment, the sequences of the nucleotide lines (1) are random sequences, for instance random sequences obtained via *in situ* synthesis, using a mixture of the 4 nucleotide precursors. As is known to the person skilled in the art, the reaction of a given sequence depends on the stringency of the reaction. The probability of cross-reaction depends primarily on sequence homology or identity. The probability to obtain a sequence of 25 nucleotides identical to another in a random synthesis is 1/4²⁵ or 1/1x10¹⁵. In view of this very low frequency of identical sequence, random synthesis of lines (1) is feasible for the purposes of the invention.

In a preferred embodiment, the nucleotide hooks (2) or hooks of the invention are chemically synthesized before being deposited on the array and thus before being fixed onto the lines (1). Enzymatic synthesis is also possible.

In a particular embodiment of the invention, the nucleotide hooks (2) are oligonucleotides or short polynucleotides with a sequence comprised of between about 10 and about 120 nucleotides, more preferably between about 15 and about 60 nucleotides, more preferably between about 20 and about 50 nucleotides, more preferably between about 20 and about 40 nucleotides. "About" in this context means that it is possible to have 1, 2, 3 to up to 5 nucleotides more or less than being mentioned.

In a particular application, the (micro)-arrays bear nucleotide hooks (2) with a sequence of less than about 100 nucleotides or bases, but giving the same binding efficiency as long polynucleotide sequences having more than about 150 nucleotides or bases. More preferably they give the same binding efficiency as long polynucleotide sequences of more than 250 nucleotides or bases. This is possible via binding, coupling or covalent linking of the nucleotide hooks (2) to nucleotide lines (1) of at least about 20, about 40 bases or nucleotides.

In a particular embodiment, the (micro)arrays are composed of at least 4, 10, 20, 50, 100, 1000, 10000 locations having both nucleotide lines and specific nucleotide hooks for at least 4, 10, 20, 50 different target nucleotide sequences to be detected and/or quantified. Such arrays have at least 4, 10, 20, 50, 100, 1000 or 10000 spots/cm².

In a particular embodiment, the lines and the hooks sequences for different target nucleotide molecules to be detected are present on at least 4, 10, 20, 50, 100, 500 or 1000 physically different supports (being preferably beads), each support being identified by a preferably different chemical or physical features (size, magnetism,...).

In a particular embodiment the nucleotide lines (1) and/or the nucleotide hooks (2) are DNA or RNA sequences, PNA (see e.g. international patent application WO0075372) or INA.

In an embodiment of the invention, the target sequences to detect, (i.e. polynucleotides or nucleotide sequences (4)) are prior to capture onto the array multiplied, amplified or copied via known genetic amplification method such as for instance a PCR method.

In a preferred embodiment, an extremity (5' or 3') of the nucleotide lines (1) is fixed onto the solid support or substrate by means of a chemical reaction.

Preferably, said nucleotide lines (1) are selected functionalized to contain or bear a specific reactive chemical function or group, which reacts with the possibly functionalized support or substrate leading to a covalent binding. The chemical function or group may be an aldehyde, amine, epoxide, acrylate, thiocyanate, N-hydroxysuccinimide..., the above list being non-exhaustive.

A preferred binding is obtained between the 5' aminated end nucleotide sequence and the aldehyde covered surface which leads to an imine bond which is then reduced in the presence of NaBH₄.

Preferably, said chemical function or group is located on either the 5' or the 3' terminal end. The nucleotide line being covalently fixed on the support; the second extremity of the nucleotide lines is activated in a second step by appropriate chemical or physical means so as to obtain chemical reactive functions for the fixation of the nucleotide hooks. The nucleotide hooks contain an appropriate group or function to be covalently coupled to nucleotide lines. The reactive group or function again may be, but is not limited to, an aldehyde, amine, epoxide, acrylate, thiocyanate, N-hydroxysuccinimide, .... Terminal end means located at the extremity of the nucleotide sequence (last base) or near the extremity being less than 50 bases and preferably less than 10 bases from the terminal base.

In another embodiment of the invention, the nucleotide lines (1) may be fixed on the array support through binding onto an adaptor. In a preferred embodiment, streptavidin is fixed onto the support and biotinylated nucleotide lines are incubated with the coated support so that binding of the lines occurred by recognition between biotin and streptavidin. Another possibility includes the use of a suitable antibody-antigen or receptor-ligand pair for obtaining an efficient binding of the nucleotide lines onto the support. Different alternatives are at the disposition of the person skilled in the art.

In a preferred embodiment, nucleotide lines (1) contain a terminal ribose which is then oxidized by periodate treatment which results in the formation of aldehyde groups. These aldehyde groups are accessible for the binding of aminated nucleotide hooks (2), coupling of both resulting in an imine bond. This imine is than reduced giving rise to a covalent amine stable link. Terminal ribose also means a ribose located close to the non bound extremity of the lines which means less than 50 bases and preferably less than 10 bases from the terminal base.

In another embodiment, solid phase synthesis via phosphoramidite methodology can be used to covalently bind the nucleotide hooks (2) to the nucleotide lines (1). In an example, the nucleotide lines are *in situ* synthesized on the surface of the solid support. The last nucleotide of the nucleotide line bears a free 5'-OH on the ribose which can covalently react with a phosphoramidite group present in position 3' of the last nucleotide of the nucleotide hook.

In a preferred embodiment of the invention, the nucleotide hooks (2) are present at a density superior to 10 fmoles and preferably 100 fmoles per cm² surface of the solid support.

In a particular embodiment of the invention, each of the specific locations used for the detection on the micro-array is covered by one particular nucleotide hook species. One hook species is preferably composed of only one molecule. However, in practice the synthesis of polynucleotides is a consecutive process performed nucleotide after nucleotide. Since each successive reaction has no 100 % yield, there will be accumulation of secondary products resulting in a heterogeneous mixture. The presence of such secondary products in the final product is not harmful to the applications of the present invention as long as the level of contamination by secondary products is not so high that it perturbs specific binding and recognition or identification of the corresponding target sequence or molecule.

An aspect of the invention concerns a solid (insoluble) support containing at given locations (6) of said solid surface (5) fixed (on the extremity of nucleotide lines) nucleotide hooks (2) specific for given target polynucleotides or nucleotide sequences (4), said nucleotide hooks (2) being fixed following their covalently link to polynucleotide lines (1) of at least 20 nucleotides long thereby forming target-specific polynucleotide sets (3), said array allowing the specific detection, identification and/or quantification of multiple, preferably at least 4 different target polynucleotides or nucleotide sequences (4) possibly present in a biological sample or test solution.

A further aspect of the invention concerns a solid (insoluble) support covered on (a limited part) of its surface (5) by nucleotides lines (1) of at least 20 nucleotides long, and at given locations (6) of this surface (5), nucleotide hooks (2) specific for given target polynucleotides or nucleotide sequences to be detected, said nucleotide hooks(2) being covalently linked to said nucleotide lines (1)thereby forming target-specific polynucleotide sets (3), said array allowing the specific detection, identification and/or quantification of multiple, preferably at least 4 different target polynucleotides or nucleotide sequences (4) possibly present in a biological sample or test solution.

A particular embodiment of the present invention relates to a solid support comprising at a first part (7) of its surface (5) a first group of at least 3 fixed polynucleotide sets (6) being spatially separated for the detection of a first group of target polynucleotide sequences (4); and at a second part (8) of the solid support surface (5) nucleotides lines (1) being unable to bind the target molecules of said first group, but being able to fix, bind or capture at spatially separated locations target specific nucleotide hooks (2) for the detection of a second group of target polynucleotide sequences (4) (see Figure 3). Preferably the second group comprises at least 2 different capture molecules, preferably at least 4 different capture molecules.
Another particular embodiment of the invention relates to a solid support which comprises at a first part (7) of its surface a first group of at least 3 fixed standard polynucleotide sequences being fixed at specific surface locations (9) not covered by nucleotide lines (1) and being spatially separated for the detection of a first group of target polynucleotide sequences(4) and at a second part (8) of the solid support surface (5) nucleotide lines (1) being unable to bind the target polynucleotide sequences of said first group, but being able to form a covalent link with target specific nucleotide hooks specific for the detection of a second group of a target polynucleotide sequences(4) (see Figure 4).

Both groups of target molecules may be present in the same sample or test solution. Preferably both groups are bound, captured and/or detected with the same efficiency. If needed, the efficiency of detection of said first and second groups is corrected in order to adequately quantify the target molecules present in the sample or test solution.

In one embodiment of the invention, the first group of polynucleotide sets is first prepared independently from the preparation and the fixation of the second group. In a particular embodiment, the first group is only present on the array support at locations not containing lines or activated lines (1). The second group of polynucleotide sets is obtained through the fixation of target-specific hooks (2) to the activated nucleotide lines (1).

Most preferably, the reagents used for attachment to the support of polynucleotide sets from the second group does not lead to the complete detachment and/or inactivation of said first group of polynucleotide sets. Preferably said detachment and/or inactivation are kept as low as possible.

In another preferred embodiment, the first group of sets are long polynucleotides and the second group of sets are composed of lines (1) and hooks (2) according to the invention, the hooks (2) being preferentially small or short target-specific polynucleotides or oligonucleotides. Preferably, the polynucleotide sets (3) are covalently bound to the support or array via reactive chemical groups or functions present on the second part of the solid support surface, which preferably is a delimitated surface of the support. Said chemical functions or groups may be activated by a chemical treatment.

Another aspect of the invention relates to a kit or a kit-in-parts for the detection, identification and/or quantification of a (micro)organism or component thereof that is possibly present in a biological sample or test solution, said kit comprising a support or a micro-array as described above.

The kit may further comprise the necessary solutions and reagents for the detection of target molecules when captured on said support or micro-array.

The kit may further comprise means and media for performing the method of the invention to obtain an array of the invention, bearing target-specific polynucleotide sets comprising target-specific nucleotide hooks linked to nucleotide lines, said capture polynucleotide sets preferably being disposed upon the surface of said solid support in the form of an array with a density of preferably at least 4 single stranded capture nucleotide sequences/cm² of solid support surface.

The arrays or supports of the invention are highly suited for the detection of different target sequences, i.e. at least one, preferably at least 4, target nucleotide sequences possibly present in a test solution or sample. Target sequences belonging to a first and a second group may be present in the same biological sample and detected simultaneously with an array according to the invention.

The invention also covers the solid support having upon at least part of its surface, nucleotide lines fixed uniformly having a sequence of at least 20 nucleotides that are fixed by a first extremity to the surface of said support and having reactive chemical groups at the other extremity that react or can be activated to react covalently with nucleotide hooks. In a particular embodiment, the arrays or supports of the invention may be used for the preparation of semi-customized arrays. Semi-customized arrays are arrays bearing a fixed number of given polynucleotide sets according to the invention or standard polynucleotide sequences of the prior art which may be supplemented in another part of the support with sequences specific for other targets of choice. Micro-arrays with standard polynucleotide sequences or sets on the first part of the array and with lines present on the second part, ready for the preparation of a second group of polynucleotide sets of choice, would then be delivered as standard product on which target-specific hooks can be spotted according to the need of the application. The present invention relates to such semi-customized arrays.

The method of the invention is particularly useful for standard production of micro-arrays having on one part of the support standard polynucleotide sets for a given number of specific target sequences, and on the other side a flexible part for the detection of different target sequences depending on the application and being non-standard. In this way, a standard array can be transformed into an indefinite number of different arrays according to the need of the user. Such array is particularly useful when long polynucleotides sets are used in the first location of the array and small oligonucleotide sequences which are easily synthesized are used on the second location. The comparison of the efficiency of binding for the two populations of polynucleotide sets allows comparison of the initial amount of each of the target molecules present in the test solution or sample. Preferably, appropriated standards are added to the test solution or sample to allow correction of the efficiency of binding of both groups of polynucleotide sets.

Another aspect of the invention covers the array support or substrate on which are bound on one part or at a fist part capture molecules for the detection of given target molecules, and on another part lines, possibly activated for the binding of hooks, adaptable for the detection of other target molecules.

The arrays of the invention are highly suited for detection and/or quantification of genes expressed by cells, possibly after copying the target gene sequences at least in part into a cDNA strand and hybridization of the cDNA onto specific capture molecules provided according to the invention. The method of the invention is particularly suited for the detection of cDNA sequences which are homologous to each other which requires a very sensitive method. Discrimination between homologous or nearly identical sequences is possible via the use of small or short specific sequences which allow such discrimination while having a good binding efficiency. The method of the invention allows discrimination between target sequences that differ in one or a few nucleotides only and that thus contain one or a few SNPs (single nucleotide polymorphisms).

Advantageously, the method and arrays of the invention can be used for the identification and/or quantification of DNA sequences, single or double stranded sequences, either obtained directly from an organism or part thereof - i.e. without any prior amplification step - or, alternatively, after amplification of part of its genomic DNA or RNA. Amplification of target sequences may be achieved via any method known in the art, including but not limited to PCR, LCR, NASBA, rolling circle or any other method which allows the production of a rather reliable copy of the given sequence.

Detection of the target sequences captured on the micro-array can be done via any physical or chemical detection method known in the art including but not limited to the use of fluorescence, colorimetry, bioluminescence, chemiluminescence, electric, surface plasmon resonance, electromagnetic signals...

The solid support or the substrate for the construction of the micro-array according to the invention preferably is selected from the group consisting of glasses, electronic devices, silicon supports, silica, metals or mixture thereof prepared in a format selected from the group of slides, discs, gel layers and/or beads. Beads are considered as arrays for as long as they have characteristics which allow to differentiate them from each other, so that identification of the beads is correlated with the identification of a given hook sequence and so of the target sequences. The above examples are not exhaustive.

In an embodiment of the invention, detection, identification and/or quantification of captured target sequences is facilitated by the use of an apparatus comprising at least a detection and/or quantification device to detect and/or quantify a signal formed at the location where a target sequence from a (micro)organism is captured on the array, possibly a reading device for information recorded upon a surface of said solid support, a computer program for recognizing the discrete regions bearing the bound target sequences upon its corresponding polynucleotide sets and their locations, possibly a quantification program of the signal present at the locations and a program for correlating the presence of the signal at these location with the diagnostic and/or the quantification of the said (micro)organism or component. The present invention extends to an apparatus adapted for these purposes and able to read the micro-array of the invention and interpret its results.

### Examples

### Example 1: Nucleotide lines fixation on a support Functionalization of glass support

The glass slides are functionalized for the presence of aldehydes according to the method described in European patent application EP1184349.

### Fixation of nucleotide lines on the support

Fixation of aminated lines on the aldehyde support are done as follows. DNA nucleotides lines that possess an amino group at the 5' end and a ribonucleotide at the 3' end are synthesized by chemical synthesis (Eurogentec, Liege, Belgium). They are diluted to 1mg/l in milliQ pure water. Each biochip is incubated at room temperature during 60 min under agitation (+/- 200 rpm) in a silver green tube containing the DNA solution. The slides are washed at room temperature: 3 times during 2 min with a 0.2% SDS solution in water, 2 times during 2 min with pure water, 1 time 5 min during min with sodium borohydride solution (2.5 mg/ml NaBH₄ in PBS /absolute ethanol ratio of 75/25) and 1 time 2 min with pure water. The slides are then washed during 3 min with pure water at 95°C. The slides are dried at room temperature before storage at 4°C.

### Example 2: nucleotide hook fixation on specific locations of the support

### Activation of functionalized lines on the support

The ribonucleotide present on the DNA nucleotide lines are oxidized into aldehyde group in the following way. The slides are incubated with the solution containing 0.1M pH 5.2 sodium acetate solution (CH₃COONa . 3H₂O, Merck, ref.-1.06267) and 25µl of a 0.45M sodium periodate solution (NaIO₄, Sigma, ref.-S1878) freshly prepared. The solution is incubated on the support at room temperature during 2h in the dark. The slides are washed with 1ml acetate buffer solution and then kept in the oxidized form at 4°C until use.

### Hook immobilization

Nucleotides hooks aminated at the 5' end are chemically synthesized by Eurogentec (Liege, Belgium). They are diluted in spotting solution (EAT, Namur, Belgium). The DNA solutions are spotted with an arrayer using plain pins on the support. The slides are dried 1h at room temperature. The slides are washed 2 times 2 min with washing buffer and 3 times with water. The slides are kept at 4°C in an evergreen tube until use. The aminated nucleotides are designed to be specific of 3 rat genes and have the following sequences:
SEQ ID N° 1 (Hook 1): Rat Fas antigen ligand (accession N°:U03470)
   5'-ataaagttttgggctgctgtgtggcaatgcagaggcaaagagaaggaact-3'
SEQ ID N° 2 (Hook 2): Rat uncoupling protein 2 (accession N°:AB010743)
   5'-atgccattgtcaactgtactgagctggtgacctatgacctcatcaaagat-3'
SEQ ID N° (Hook 3): Rat ribosomal protein L19 (accession N°:J02650)
   5'-cgtcctccgctgtggtaaaaagaaggtgtggttggaccccaatgaaacca-3'

### Example 3: detection of cDNA on biochips by colorimetry

The surface of the support containing the capture molecules are surrounding with an hybridization frame which delimits the surface of the support being in contact with the solution containing the target sequences. The array is incubated with biotinylated cDNA from rat liver as explained by Delongueville et al. 2002 (Biochem. Pharmacol. 64:137-149). After hybridization of the target DNA, the arrays are washed and incubated at room temperature during 45 min in an Evergreen tube containing 15 ml anti-biotin antibody coupled to a 20 nm gold particle (British Biocell International, ref. BL-GAB20) diluted 1/100 in a blocking buffer (Eppendorf Hamburg, Germany). The biochips are then washed 5 times during 2 min with B1 buffer (Eppendorf Hamburg, Germany). Each biochip is incubated in a new Eppendorf tube at room temperature in the dark during 10 min in Silverquant solution (Eppendorf, Hamburg, Germany). The revelation is stopped by washing 2 times with 700 µl milliQ water. The biochips are then scanned and quantified in a colorimetric Scanarray (Eppendorf, Hamburg, Germany). After digitalization of the picture, a software program (Eppendorf, Hamburg, Germany) is used in order to delimitate the spot surface, integrate the signal for each spot, subtract the local background around each spot, identify the localization of the spots and correlate the localization with the identity of the target. Quantification is obtained by comparison of the signal obtained with spiked internal standards and the signals of the different target nucleotide spots.

### Example 4: detection of cDNA on biochips by fluorescence

The experiment is essentially performed as in example 3 but after hybridization with the biotinylated target cDNA, the arrays are incubated with anti-Cyanine antibody (Jackson ImmunoResearch, Cy3: ref.-200.162.096, Cy5: ref.-200.172.096) diluted 1/1000 in a blocking buffer. The biochips are then washed 4 times during 2 min with B1 buffer. The biochips are dried at room temperature and scanned with GMS418 ScanArray (General Scanning). After digitalization of the picture, the imagene software (Biodiscovery, Marina Del Rey, USA) is used in order to delimitate the spot surface, integrate the signal for each spot, subtract the local background around each spot, identify the localization of the spots and correlate the localization with the identity of the target sequences. The quantification of the target sequences present in the sample is obtained essentially as described in the publication of Delongueville *et al*. 2002 (Biochem. Pharmacol. 64:137-149).

## Claims

1. A method for the construction of micro-arrays of polynucleotide sets (3) on a surface (5) of a solid support to be used for the detection and/or the quantification of at least four different target polynucleotides or nucleotide sequences (4), possibly present in a biological sample or test solution, said method comprising the steps of :
- fixing nucleotide lines sequences (1) upon a surface (5) of the solid support, said nucleotide lines (1) being at least 20 nucleotides long and having a random nucleotide sequence;
- depositing in at least 4 specific locations (6) on the surface (5) of said solid support, nucleotide hooks (2) having a sequence specific for one of said at least four different target polynucleotides sequences (4) to be detected and/or quantified and;
- covalently linking at said specific surface locations (6) the nucleotide hooks (2) to the nucleotide line sequences (1) in order to form polynucleotide sets (3), specific for the binding of the said target polynucleotide sequences (4).

2. The method according to claim 1, wherein the surface (5) of the solid surface used for the construction of micro-arrays is uniformly covered with nucleotides lines (1) sequences.

3. The method according to claims 1 to 2, wherein the nucleotide lines (1) sequences are covalently fixed on the surface of the solid support by one of their extremities (5', 3') and are covalently fixed to the nucleotide hooks (2) by the other extremity.

4. The method according to claims 1 to 3, wherein the solid surface comprises a micro-array of at least four spots of polynucleotide sets (3) per cm²

5. The method according to claims 1 to 4 wherein the sequence of the nucleotide lines (1) contains less than 15 bases, preferably less than 10, and still preferably less than 5 contiguous bases complementary to the target polynucleotide sequences (4) to be detected.

6. The method according to any of the preceding claims wherein the sequences of the nucleotide lines (1) are multiple random sequences.

7. The method according to any of the preceding claims, wherein all nucleotide lines (1) consist of the same random sequence.

8. The method according to any of the preceding claims wherein the nucleotide hooks (2) have a sequence comprised of between about 10 and about 120 nucleotides.

9. The method according to any of the preceding claims, wherein the nucleotide hooks (2) are chemically synthesized oligonucleotides.

10. The method according to any of the preceding claims, wherein the nucleotide lines (1) are synthesized in situ upon the surface (5) of the solid support.

11. The method according to any of the preceding claims, wherein the nucleotide lines (1) coupled to nucleotide hooks (2) are sequences having different binding affinities for different target polynucleotide sequences (4) and are attached to different solid supports, each of these solid supports being **characterized by** a specific chemical or physical feature.

12. The method according to claim 11, wherein the solid support comprises different beads of different chemical or physical features.

13. The method according to any of the preceding claims, wherein the target polynucleotide sequences (4) are amplified or copied nucleotide sequences.

14. A solid support comprising nucleotide lines (1) being at least 20 nucleotides long and having a random nucleotide sequence being fixed by one of their extremities (3' or 5'), uniformly upon at least one surface (5) of the solid support.

15. The solid support according to the claim 14, wherein all of the nucleotide lines (1) consist of the same random sequence.

16. The solid support according to the claim 14, wherein the sequence of the nucleotide lines (1) are multiple random sequences.

17. The solid support according to any of the preceding claims 14 to 16, wherein the extremity (5' or 3') of the nucleotide line sequence (1) which is not bound to the surface (5) of the solid support comprises a reactive group or function able to create a covalent link with the extremity of another nucleotide sequence (2).

18. The solid support according to the claim 17 wherein the reactive chemical group present at the extremity of the nucleotide lines sequence is selected from the group consisting of aldehyde, epoxide and acrylate group.

19. The solid support according to claims 14 to 18 wherein the nucleotide line sequence comprises at least one nucleotide-ribose at (or near) its non bound extremity.

20. The solid support according to claims 14 to 19, wherein the density of the nucleotide lines (1) bound to the solid support at a specific location is superior than 10 fmoles, preferably 100 fmoles per cm² of solid support surface.

21. The solid support according to any of the preceding claims 14 to 20, wherein nucleotide lines (1) sequence bound at specific locations (6) upon the solid support surface (5) present a covalent link with nucleotide hooks (2) in order to form polynucleotide sets (3) at said specific locations (6) of the solid support surface (5), said nucleotide hooks (2) having a sequence specific of one target polynucleotide sequence (4) to be detected and/or quantified and wherein the covalent link between the extremity of the nucleotide lines (1) and the extremity of the nucleotide hooks (2) is not a phosphodiester link.

22. The solid support according to the claim 21, which comprises at a first part (7) of its surface (5) a first group of at least 3 fixed polynucleotide sets (3) being spatially separated for the detection of a first group of target polynucleotide sequences(4) and at a second part (8) of the solid support surface (5) nucleotide lines (1) being unable to bind the target polynucleotide sequences of said first group, but being able to form a covalent link with target specific nucleotide hooks specific for the detection of a second group of a target polynucleotide sequences(4).

23. The solid support according to the claim 21, which comprises at a first part (7) of its surface a first group of at least 3 fixed standard polynucleotide sequences being fixed at specific surface locations (9) not covered by nucleotide lines (1) and being spatially separated for the detection of a first group of target polynucleotide sequences(4) and at a second part (8) of the solid support surface (5) nucleotide lines (1) being unable to bind the target polynucleotide sequences of said first group, but being able to form a covalent link with target specific nucleotide hooks specific for the detection of a second group of a target polynucleotide sequences(4).

24. The solid support according to any of the preceding claims 14 to 23, which is selected from the group consisting of glasses, electronic devices, silicon supports, plastic supports, silica support, metal supports or a mixture thereof.

25. The solid support according to any of the preceding claims 14 to 24, wherein said solid support is in a format selected from the group consisting of slides, dics, gel layers and micro beads.

26. The solid support according to any of the preceding claims 21 to 25, wherein the density of the polynucleotide sets (3) bound to the solid support at a specific location is superior than 10 fmoles, preferably 100 fmoles per cm² of solid support surface.

27. A kit of parts for the detection, identification and/or quantification of a micro organism or nucleotide component thereof that is possibly present in a biological sample or test solution, said kit comprising the solid support according to any of the preceding claims 14 to 26 and means and media for the detection of target polynucleotide sequences (4) obtained from said microorganisms or nucleotide component.

28. The kit according to the claim 27, which further comprises nucleotide hooks sequences (2) specific for one or more different target polynucleotides sequences (4) to be detected and/or quantified and possibly reagents for allowing the formation of a covalent link between the extremity of the nucleotide hooks (2) sequence and the extremity of the nucleotide lines (1) sequence.

29. An apparatus for the detection, identification and/or quantification of micro organisms or a nucleotide component thereof possibly present in a biological sample or test solution which comprises the solid support according to any of the preceding claims 14 to 26, possibly present in a kit of parts according to the claim 27 or 28, said apparatus further comprising a detection and/or quantification device able to detect and/or quantify a signal formed at a location where a target nucleotide sequence (4) is bound to a polynucleotide sets (3) of the solid support surface (5), possibly a reading device for reading the information recorded upon the surface (5) of the solid support, and a computer program for detecting the discrete regions bearing target polynucleotides sequences (4) bound to their corresponding polynucleotide sets (3) and their locations for correlating the presence of a detected signal at these locations with the diagnosis and/or the quantification of the micro organisms or the nucleotide component thereof.

30. Use of the kit or apparatus according to any of the preceding claims 27 to 29 for diagnosis and gene expression analysis.

31. The use of claim 30 for the identification, detection and/or quantification of multiple different target of polynucleotides, possibly obtained by genetic amplification of copy steps, and being obtained from genetic sequences that belong to different genetic taxonomic groups such as class, family, genus, species or individuals.

## Patentansprüche

1. Verfahren für die Konstruktion von Mikro-Arrays aus Polynukleotidsätzen (3) auf einer Oberfläche (5) eines festen Trägers, die für die Detektion und/oder Quantifizierung mindestens vier verschiedener Ziel-Polynukleotide oder -Nukleotidsequenzen (4) verwendet werden, die eventuell in einer biologischen Probe oder Testlösung vorhanden sind, wobei das Verfahren folgende Schritte umfasst:
- Fixieren von Nukleotidliniensequenzen (1) auf einer Oberfläche (5) des festen Trägers, wobei die Nukleotidlinien (1) mindestens 20 Nukleotide lang sind und eine zufällige Nukleotidsequenz haben;
- Auftragen an mindestens 4 spezifischen Stellen (6) auf der Oberfläche (5) des festen Trägers von Nukleotidhaken (2) mit einer Sequenz, die für eine der mindestens vier verschiedenen Ziel-Polynukleotidsequenzen (4), die detektiert und/oder quantifiziert werden soll, spezifisch ist; und
- kovalente Bindung an den spezifischen Oberflächenstellen (6) der Nukleotidhaken (2) an die Nukleotidliniensequenzen (1), um Polynukleotidsätze (3) zu bilden, die für die Bindung der Ziel-Polynukleotidsequenzen (4) spezifisch sind.

2. Verfahren nach Anspruch 1, wobei die Oberfläche (5) der festen Oberfläche, die für die Konstruktion von Mikro-Arrays verwendet wird, gleichförmig mit Nukleotidlinien- (1) Sequenzen bedeckt ist.

3. Verfahren nach den Ansprüchen 1 bis 2, wobei die Nukleotidlinien- (1) Sequenzen kovalent an der Oberfläche des festen Trägers durch eines ihrer Enden (5', 3') fixiert sind, und mit dem anderen Ende kovalent an den Nukleotidhaken (2) fixiert sind.

4. Verfahren nach den Ansprüchen 1 bis 3, wobei die feste Oberfläche einen Mikro-Array von mindestens vier Stellen von Polynukleotidsätzen (3) pro cm² umfasst.

5. Verfahren nach den Ansprüchen 1 bis 4, wobei die Sequenz der Nukleotidlinien (1) weniger als 15 Basen, vorzugsweise weniger als 10 und insbesondere weniger als 5 angrenzende Basen enthält, die zu den zu detektierenden Ziel-Polynukleotidsequenzen (4) komplementär sind.

6. Verfahren nach irgendeinem der vorangehenden Ansprüche, wobei die Sequenzen der Nukleotidlinien (1) mehrfache zufällige Sequenzen sind.

7. Verfahren nach irgendeinem der vorangehenden Ansprüche, wobei alle Nukleotidlinien (1) aus derselben zufälligen Sequenz bestehen.

8. Verfahren nach irgendeinem der vorangehenden Ansprüche, wobei die Nukleotidhaken (2) eine Sequenz haben, die aus etwa 10 bis etwa 120 Nukleotiden besteht.

9. Verfahren nach irgendeinem der vorangehenden Ansprüche, wobei die Nukleotidhaken (2) chemisch synthetisierte Oligonukleotide sind.

10. Verfahren nach iregndeinem der vorangehenden Ansprüche, wobei die Nukleotidlinien (1) *in situ* auf der Oberfläche (5) des festen Trägers synthetisiert sind.

11. Verfahren nach irgendeinem der vorangehenden Ansprüche, wobei die Nukleotidlinien (1), die an Nukleotidhaken (2) gekoppelt sind, Sequenzen mit verschiedenen Bindungsaffinitäten für verschiedene Ziel-Polynukleotidsequenzen (4) sind und an verschiedene feste Träger angeheftet sind, wobei jeder dieser festen Träger durch ein spezifisches chemisches oder physikalisches Merkmal **gekennzeichnet** ist.

12. Verfahren nach Anspruch 11, wobei der feste Träger verschiedene Kügelchen mit verschiedenen chemischen oder physikalischen Merkmalen umfasst.

13. Verfahren nach irgendeinem der vorangehenden Ansprüche, wobei die Ziel-Polynukleotidsequenzen (4) amplifizierte oder kopierte Nukleotidsequenzen sind.

14. Fester Träger, umfassend Nukleotidlinien (1), die mindestens 20 Nukleotide lang sind und eine zufällige Nukleotidsequenz haben, die durch eines ihrer Enden (3' oder 5') gleichförmig auf mindestens einer Oberfläche (5) des festen Trägers fixiert sind.

15. Fester Träger nach Anspruch 14, wobei alle Nukleotidlinien (1) aus derselben zufälligen Sequenz bestehen.

16. Fester Träger nach Anspruch 14, wobei die Sequenzen der Nukleotidlinien (1) mehrfache zufällige Sequenzen sind.

17. Fester Träger nach irgendeinem der vorangehenden Ansprüche 14 bis 16, wobei das Ende (5' oder 3') der Nukleotidliniensequenz (1), das nicht an die Oberfläche (5) des festen Trägers gebunden ist, eine reaktive Gruppe oder Funktion umfasst, die zur Bildung einer kovalenten Bindung mit dem Ende einer anderen Nukleotidsequenz (2) imstande ist.

18. Fester Träger nach Anspruch 17, wobei die reaktive chemische Gruppe, die an dem Ende der Nukleotidliniensequenz vorhanden ist, ausgewählt ist aus der Gruppe bestehend aus Aldehyd-, Epoxid- und Acrylatgruppe.

19. Fester Träger nach den Ansprüchen 14 bis 18, wobei die Nukleotidliniensequenz mindestens eine Nukleotid-Ribose an (oder nahe) ihrem ungebundenen Ende umfasst.

20. Fester Träger nach den Ansprüchen 14 bis 19,, wobei die Dichte der Nukleotidlinien (1), die an den festen Träger an einer spezifischen Stelle gebunden sind, höher als 10 fmol, vorzugsweise 100 fmol pro cm² der Oberfläche des festen Trägers ist.

21. Fester Träger nach irgendeinem der vorangehenden Ansprüche 14 bis 20, wobei Nukleotidlinien- (1) Sequenzen, die an spezifischen Stellen (6) auf der Oberfläche (5) des festen Trägers gebunden sind, eine kovalente Bindung mit Nukleotidhaken (2) aufweisen, um Polynukleotidsätze (3) an den spezifischen Stellen (6) der Oberfläche (5) des festen Trägers zu bilden, wobei Nukleotidhaken (2) eine Sequenz haben, die für eine zu detektierende und/oder quantifizierende Ziel-Polynukleotidsequenz (4) spezifisch ist, und wobei die kovalente Bindung zwischen dem Ende der Nukleotidlinien (1) und dem Ende der Nukleotidhaken (2) keine Phosphodiesterbindung ist.

22. Fester Träger nach Anspruch 21, der an einem ersten Teil (7) seiner Oberfläche (5) eine erste Gruppe von mindestens 3 fixierten Polynukleotidsätzen (3) umfasst, die räumlich getrennt sind, für die Detektion einer ersten Gruppe von Ziel-Polynukleotidsequenzen (4), und an einem zweiten Teil (8) der Oberfläche (5) des festen Trägers Nukleotidlinien (1), die nicht imstande sind, die Ziel-Polynukleotidsequenzen der ersten Gruppe zu binden, sondern imstande sind, eine kovalente Bindung mit zielspezifischen Nukleotidhaken zu bilden, die für die Detektion einer zweiten Gruppe von Ziel-Polynukleotidsequenzen (4) spezifisch sind.

23. Fester Träger nach Anspruch 21, der an einem ersten Teil (7) seiner Oberfläche eine erste Gruppe von mindestens 3 fixierten Standard-Polynukleotidsequenzen umfasst, die an spezifischen Oberflächestellen (9) fixiert sind, die nicht von Nukleotidlinien (1) bedeckt sind und räumlich getrennt sind, für die Detektion einer ersten Gruppe von Ziel-Polynukleotidsequenzen (4), und an einem zweiten Teil (8) der Oberfläche (5) des festen Trägers Nukleotidlinien (1), die nicht imstande sind, die Ziel-Polynukleotidsequenzen der ersten Gruppe zu binden, sondern imstande sind, eine kovalente Bindung mit zielspezifischen Nukleotidhaken zu bilden, die für die Detektion einer zweiten Gruppe von Ziel-Polynukleotidsequenzen (4) spezifisch sind.

24. Fester Träger nach irgendeinem der vorangehenden Ansprüche 14 bis 23, der ausgewählt ist aus der Gruppe bestehend aus Glas, elektronischen Vorrichtungen, Siliziumträgern, Kunststoffträgern, Silikaträgern, Metallträgern oder einer Mischung davon.

25. Fester Träger nach irgendeinem der vorangehenden Ansprüche 14 bis 24, wobei der feste Träger in einem Format ist, das ausgewählt ist aus der Gruppe bestehend aus Objektträgern, Scheiben, Gelschichten und Mikrokügelchen.

26. Fester Träger nach irgendeinem der vorangehenden Ansprüche 21 bis 25, wobei die Dichte der Polynukleotidsätze (3), die an den festen Träger an einer spezifischen Stelle gebunden sind, höher als 10 fmol, vorzugsweise 100 fmol pro cm² der Oberfläche des festen Trägers ist.

27. Kit von Komponenten für die Detektion, Identifizierung und/oder Quantifizierung eines Mikroorganismus oder einer Nukleotidkomponente davon, der/die eventuell in einer biologischen Probe oder Testlösung vorhanden ist, wobei der Kit den festen Träger nach einem der vorangehenden Ansprüche 14 bis 26 umfasst, sowie Mittel und Medien für die Detektion von Ziel-Polynukleotidsequenzen (4), die von den Mikroorganismen oder der Nukleotidkomponente erhalten werden.

28. Kit nach Anspruch 27, der des Weiteren Nukleotidhakensequenzen (2) umfasst, die für eine oder mehrere verschiedene zu detektierende und/oder quantifizierende Ziel-Polynukleotidsequenzen (4) spezifisch sind, und eventuell Reagenzien, die die Bildung einer kovalenten Bindung zwischen dem Ende der Nukleotidhaken- (2) Sequenz und dem Ende der Nukleotidlinien- (1) Sequenz ermöglichen.

29. Gerät für die Detektion, Identifizierung und/oder Quantifizierung von Mikroorganismen oder einer Nukleotidkomponente davon, die eventuell in einer biologischen Probe oder Testlösung vorhanden sind, das den festen Träger nach irgendeinem der vorangehenden Ansprüche 14 bis 26 umfasst, der eventuell in einem Kit von Komponenten nach Anspruch 27 oder 28 vorhanden ist, wobei das Gerät des Weiteren eine Detektions- und/oder Quantifizierungsvorrichtung umfasst, die imstande ist, ein Signal zu detektieren und/oder zu quantifizieren, das an einer Stelle gebildet wird, wo eine Ziel-Nukleotidsequenz (4) an einen Polynukleotidsatz (3) der Oberfläche (5) des festen Trägers gebunden ist, sowie eventuell eine Lesevorrichtung zum Lesen der Informationen, die auf der Oberfläche (5) des festen Trägers aufgezeichnet sind, und ein Computer-Programm zum Detektieren der einzelnen Bereiche, die Ziel-Polynukleotidsequenzen (4) tragen, die an ihre entsprechenden Polynukleotidsätze (3) gebunden sind, und deren Stellen zum Korrelieren der Gegenwart eines an diesen Stellen detektierten Signals mit der Diagnose und/oder der Quantifizierung der Mikroorganismen oder deren Nukleotidkomponente.

30. Verwendung des Kits oder Geräts nach irgendeinem der vorangehenden Ansprüche 27 bis 29 zur Diagnose und Genexpressionsanalyse.

31. Verwendung nach Anspruch 30 für die Identifizierung, Detektion und/oder Quantifizierung mehrfacher verschiedener Ziele von Polynukleotiden, die eventuell durch Genamplifizierung von Kopierschritten erhalten werden, und von genetischen Sequenzen erhalten werden, die zu verschiedenen genetischen taxonomischen Gruppen, wie Klasse, Familie, Gattung, Spezies oder Individuen, gehören.

## Revendications

1. Procédé pour la construction de microréseaux de jeux de polynucléotides (3) sur une surface (5) d'un support solide pour la détection et/ou la quantification d'au moins quatre polynucléotides ou séquences nucléotidiques cibles (4) différents, éventuellement présent(e)s dans un échantillon biologique ou une solution d'essai, ledit procédé comprenant les étapes suivantes :
- la fixation de séquences de lignes de nucléotides (1) sur une surface (5) du support solide, lesdites lignes de nucléotides (1) ayant une longueur d'au moins 20 nucléotides et une séquence nucléotidique aléatoire;
- le dépôt à au moins 4 emplacements spécifiques (6), sur la surface (5) dudit support solide, de crochets de nucléotides (2) ayant une séquence spécifique pour l'une desdites au moins quatre séquences polynucléotidiques cibles (4) différentes à détecter et/ou à quantifier; et
- la liaison par covalence, auxdits emplacements de surface spécifiques (6), des crochets de nucléotides (2) avec les séquences de lignes de nucléotides (1), afin de former des jeux de polynucléotides (3) spécifiques à la liaison desdites séquences polynucléotidiques cibles (4).

2. Procédé selon la revendication 1, dans lequel la surface (5) de la surface solide utilisée pour la construction de microréseaux est uniformément recouverte de séquences de lignes de nucléotides (1).

3. Procédé selon les revendications 1 à 2, dans lequel les séquences de lignes de nucléotides (1) sont fixées par covalence, à la surface du support solide, par une de leurs extrémités (5', 3') et sont fixées par covalente aux crochets de nucléotides (2) par l'autre extrémité.

4. Procédé selon les revendications 1 à 3, dans lequel la surface solide comprend un microréseau d'au moins quatre taches de jeux de polynucléotides (3) par cm².

5. Procédé selon les revendications 1 à 4, dans lequel la séquence de lignes de nucléotides (1) contient moins de 15 bases, de préférence, moins de 10, et, mieux encore, moins de 5 bases contiguës complémentaires aux séquences polynucléotidiques cibles (4) à détecter.

6. Procédé selon l'une quelconque des revendications précédentes, dans lequel les séquences de lignes de nucléotides (1) sont des séquences aléatoires multiples.

7. Procédé selon l'une quelconque des revendications précédentes, dans lequel toutes les lignes de nucléotides (1) sont constituées de la même séquence aléatoire.

8. Procédé selon l'une quelconque des revendications précédentes, dans lequel les crochets de nucléotides (2) ont une séquence comprise entre environ 10 et environ 120 nucléotides.

9. Procédé selon l'une quelconque des revendications précédentes, dans lequel les crochets de nucléotides (2) sont des oligonucléotides synthétisés chimiquement.

10. Procédé selon l'une quelconque des revendications précédentes, dans lequel les lignes de nucléotides (1) sont synthétisées *in situ* sur la surface (5) du support solide.

11. Procédé selon l'une quelconque des revendications précédentes, dans lequel les lignes de nucléotides (1) couplées aux crochets de nucléotides (2) sont des séquences ayant différentes affinités de liaison pour différentes séquences polynucléotidiques cibles (4) et sont fixées sur différents supports solides, chacun de ces supports solides étant **caractérisé par** une propriété chimique ou physique spécifique.

12. Procédé selon la revendication 11, dans lequel le support solide comprend différentes billes aux propriétés chimiques ou physiques différentes.

13. Procédé selon l'une quelconque des revendications précédentes, dans lequel les séquences polynucléotidiques cibles (4) sont des séquences nucléotidiques amplifiées ou copiées.

14. Support solide comprenant des lignes de nucléotides (1) ayant une longueur d'au moins 20 nucléotides et ayant une séquence nucléotidique aléatoire, qui sont fixées, par l'une de leurs extrémités (3' ou 5'), de manière uniforme, sur au moins une surface (5) du support solide.

15. Support solide selon la revendication 14, dans lequel toutes les lignes de nucléotides (1) sont constituées de la même séquence aléatoire.

16. Support solide selon la revendication 14, dans lequel les séquences des lignes de nucléotides (1) sont des séquences aléatoires multiples.

17. Support solide selon l'une quelconque des revendications précédentes 14 à 16, dans lequel l'extrémité (5' ou 3') de la séquence de lignes de nucléotides (1), qui n'est pas liée à la surface (5) du support solide, comprend un groupement réactif ou une fonction réactive capable de créer une liaison covalente avec l'extrémité d'une autre séquence nucléotidique (2).

18. Support solide selon la revendication 17, dans lequel le groupement chimique réactif présent à l'extrémité de la séquence de lignes de nucléotides est choisi dans le groupe constitué des groupements aldéhyde, époxyde et acrylate.

19. Support solide selon les revendications 14 à 18, dans lequel la séquence de lignes de nucléotides comprend au moins un nucléotide-ribose à son extrémité non liée (ou à proximité de celle-ci) .

20. Support solide selon les revendications 14 à 19, dans lequel la densité des lignes de nucléotides (1), liées au support solide à un emplacement spécifique, est supérieure à 10 fmoles, de préférence 100 fmoles par cm² de surface de support solide.

21. Support solide selon l'une quelconque des revendications précédentes 14 à 20, dans lequel la séquence de lignes de nucléotides (1), liée à des emplacements spécifiques (6) sur la surface de support solide (5), présente une liaison covalente avec des crochets de nucléotides (2) pour former des jeux de polynucléotides (3) auxdits emplacements spécifiques (6) de la surface de support solide (5), lesdits crochets de nucléotides (2) ayant une séquence spécifique à une séquence polynucléotidique cible (4) à détecter et/ou à quantifier, et dans lequel la liaison covalente entre l'extrémité des lignes de nucléotides (1) et l'extrémité des crochets de nucléotides (2) n'est pas une liaison phosphodiester.

22. Support solide selon la revendication 21, qui comprend, sur une première partie (7) de sa surface (5), un premier groupement d'au moins 3 jeux de polynucléotides (3) fixés, séparés spatialement pour la détection d'un premier groupement de séquences polynucléotidiques cibles (4) et, sur une seconde partie (8) de la surface du support (5), des lignes de nucléotides (1) incapables de lier les séquences polynucléotidiques cibles dudit premier groupement, mais capables de former une liaison covalente avec des crochets de nucléotides spécifiques cibles spécifiques pour la détection d'un deuxième groupement de séquences polynucléotidiques cibles (4).

23. Support solide selon la revendication 21, qui comprend, sur une première partie (7) de sa surface, un premier groupement d'au moins 3 séquences polynucléotidiques standard fixées à des emplacements de surface spécifiques (9) non couverts par des lignes de nucléotides (1) et séparés spatialement pour la détection d'un premier groupement de séquences polynucléotidiques cibles (4) et, sur une seconde partie (8) de la surface du support solide (5), des lignes de nucléotides (1) incapables de lier les séquences polynucléotidiques cibles dudit premier groupement, mais capables de former une liaison covalente avec les crochets de nucléotides spécifiques cibles spécifiques pour la détection d'un deuxième groupement de séquences polynucléotidiques cibles (4).

24. Support solide selon l'une quelconque des revendications 14 à 23, qui est choisi dans le groupe constitué du verre, de dispositifs électroniques, de supports de silicium, de supports plastiques, de supports de silice, de supports métalliques ou d'un mélange de ceux-ci.

25. Support solide selon l'une quelconque des revendications précédentes 14 à 24, dans lequel ledit support solide se présente dans un format choisi dans le groupe constitué de lames, de disques, de couches de gel et de microbilles.

26. Support solide selon l'une quelconque des revendications précédentes 21 à 25, dans lequel la densité des jeux de nucléotides (3), liés au support solide à un emplacement spécifique, est supérieure à 10 fmoles, de préférence à 100 fmoles par cm² de surface de support solide.

27. Trousse de pièces pour la détection, l'identification et/ou la quantification d'un microorganisme ou d'un composant nucléotidique de celui-ci, qui est éventuellement présent dans un échantillon biologique ou une solution d'essai, ladite trousse comprenant le support solide selon l'une quelconque des revendications précédentes 14 à 26 et des moyens pour la détection de séquences polynucléotidiques cibles (4) obtenues à partir desdits microorganismes ou dudit composant nucléotidique.

28. Trousse selon la revendication 27, qui comprend également des séquences de crochets de nucléotides (2) spécifiques à une ou plusieurs séquences polynucléotidiques cibles (4) différentes à détecter et/ou à quantifier et, éventuellement, des réactifs pour permettre la formation d'une liaison covalente entre l'extrémité de la séquence de crochets de nucléotides (2) et l'extrémité de la séquence de lignes de nucléotides (1).

29. Appareil pour la détection, l'identification et/ou la quantification de microorganismes ou d'un composant nucléotidique de ceux-ci, éventuellement présents dans un échantillon biologique ou une solution d'essai, qui comprend le support solide selon l'une quelconque des revendications précédentes 14 à 26, éventuellement présent dans une trousse de pièces selon la revendication 27 ou 28, ledit appareil comprenant également un dispositif de détection et/ou de quantification capable de détecter et/ou de quantifier un signal formé à un emplacement où une séquence nucléotidique cible (4) est liée à un jeu de polynucléotides (3) de la surface de support solide (5), éventuellement un dispositif de lecture pour lire les informations enregistrées à la surface (5) du support solide, et un programme informatique pour détecter les régions discrètes portant des séquences polynucléotidiques cibles (4) liées à leurs jeux de polynucléotides correspondants (3) et à leurs emplacements, afin d'établir une corrélation entre la présence d'un signal détecté à ces emplacements et le diagnostic et/ou la quantification des microorganismes ou du composant nucléotidique de ceux-ci.

30. Utilisation de la trousse ou de l'appareil selon l'une quelconque des revendications précédentes 27 à 29 pour le diagnostic et l'analyse de l'expression génétique.

31. Utilisation selon la revendication 30 pour l'identification, la détection et/ou la quantification de cibles polynucléotidiques différentes multiples, éventuellement obtenues par amplification génétique d'étapes de copie, et tirées de séquences génétiques qui appartiennent à différents groupes taxonomiques génétiques, tels que la classe, la famille, le genre, l'espèce ou les individus.


### SEQUENCE LISTING

<110> Eppendorf Array Technologies
<120> PREPARATION METHOD AND USE OF MICRO-ARRAYS FOR DETECTION OF MULTIPLE POLYNUCLEOTIDE SEQUENCES WITH HIGH SENSITIVITY
<130> BP.EAT.003A/EP
<140> EP04447111.8
   <141> 2004-05-04
<160> 3
<170> PatentIn version 3.2
<210> 1
   <211> 50
   <212> DNA
   <213> Rat Fas antigen ligand (accession N°: U03470)
<400> 1
   ataaagtttt gggctgctgt gtggcaatgc agaggcaaag agaaggaact 50
<210> 2
   <211> 50
   <212> DNA
   <213> Rat uncoupling protein 2 (accession N°: AB010743)
<400> 2
   atgccattgt caactgtact gagctggtga cctatgacct catcaaagat 50
<210> 3
   <211> 50
   <212> DNA
   <213> Rat ribosomal protein L19 (accession N°: J02650)
<400> 3
   cgtcctccgc tgtggtaaaa agaaggtgtg gttggacccc aatgaaacca 50